Europäisches Patentamt

European Patent Office

Office européen des brevets

(11) Veröffentlichungsnummer: **0 017 812**
**A1**

(19)

(12) **EUROPÄISCHE PATENTANMELDUNG**

(21) Anmeldenummer: 80101594.2

(22) Anmeldetag: 26.03.80

(51) Int. Cl.³: **G 01 N 33/52, B 01 D 9/00**

(30) Priorität: 06.04.79 DE 2913889

(43) Veröffentlichungstag der Anmeldung: 29.10.80
Patentblatt 80/22

(84) Benannte Vertragsstaaten: **AT BE CH DE FR GB IT LU NL SE**

(71) Anmelder: **Compur-Electronic GmbH, Steinerstrasse 15, D-8000 München 70 (DE)**

(72) Erfinder: **Schuler, Peter, Dr. Dipl.-Chem., Am Knie 7, D-8000 München 60 (DE)**
Erfinder: **Braun, Erwin, Unteranger 1, D-8132 Tutzing (DE)**

(74) Vertreter: **Geyer, Ulrich F., Dr. Dipl.-Phys., St.-Anna-Strasse 15, D-8000 München 22 (DE)**

(54) Verfahren zur kristallinen Abscheidung von Chromogenen.

(57) Um eine homogene kristalline und gut reproduzierbare Abscheidung von Chromogenen in Kapillaren, insbesondere für die Serienfertigung zu erzielen wird die Auskristallisierung des Chromogens aus einer Lösung zunächst durch eine relativ starke Übersättigung ausgelöst und läuft danach bei einer demgegenüber gering übersättigten bzw. gesättigten oder nicht übersättigten Lösung ab. Die Verdunstungsgeschwindigkeit wird beispielsweise durch die Wahl der Lösungstemperatur und/oder eines Lösungsmittelgemisches aus einer das Chromogen lösenden und einer das Chromogen nicht lösenden Komponente so gewählt, dass die Lösung in einen zwar gesättigten, eine langsame Kristallisierung ermöglichenden Zustand, nicht aber in einen stark übersättigten Zustand gelangt. Dadurch steht ausreichend Zeit für eine ungehinderte Kristallisation zur Verfügung, so dass sich das Chromogen nicht als amorphes Öl, sondern in kristalliner Form und ohne Pfropfenbildung abscheidet, wodurch die Saugfähigkeit der Kapillare erhalten bleibt.

## Beschreibung

Die Erfindung betrifft ein Verfahren zur kristallinen Abscheidung von Chromogenen, insbesondere für klinisch-chemische Bestimmungen.

Bei der Konzentrationsbestimmung von Stoffwechselparametern aus Körperflüssigkeiten im Bereich der klinischen Chemie spielt die exakte Volumendosierung von Probe und in Lösung befindlichen Reagenzien eine entscheidende Rolle. Zumeist bedient man sich dabei Dosierhilfen im Sinne von Pipetten, die ihrem Wesen nach Glas- oder Kolbenhubpipetten bzw. voll- oder teilautomatisierte Dosiereinrichtungen sein können. Diese Hilfsmittel haben den Nachteil eines hohen Preises, sie bedingen außerdem eine fortlaufende Überwachung im Sinne einer Eichkontrolle, um die Richtigkeit des dosierten Flüssigkeitsvolumens sicherzustellen. Bei teilautomatischen und manuell betätigten Systemen ist außerdem die Möglichkeit von Bedienungsfehlern nicht auszuschließen, so daß die Bedienung durch ungeschultes Personal ausscheidet.

Es ist deshalb mit Erfolg versucht worden, die geschilderten Schwierigkeiten dadurch zu umgehen, daß man die einzelnen Komponenten des Reagenzgemisches nach Maßgabe der sich ergebenden Haltbarkeit entweder in reiner Form oder als Teilgemisch vorportioniert und dem Anwender damit Pipettierschritte erspart. Zur Dosierung der Probe (Blut, Serum oder Plasma) kann zusätzlich eine sog. End-To-End-Kapillare verwendet werden, die sich aufgrund der Kapillarkräfte selbsttätig füllt, womit die Gefahr von Dosierfehlern ausgeschaltet oder zumindest verringert werden kann.

Ist eine Reagenzkomponente oder ein Gemisch aus mehreren Komponenten in Lösung stabil, so kann die Vorportionierung durch Vorgabe eines maschinell abgefüllten Flüssigkeitsvolumens erfolgen. Dazu eignet sich z.B. die Pufferlösung, die vorteilhafterweise gleich in die Meßküvette abgefüllt werden kann. Ein solches Verfahren, das sich zusätzlich einer Probenkapillare bedient, ist in der DE-OS 24 22 260 beschrieben.

Eine Vorportionierung bereitet jedoch Schwierigkeiten galenischer Art, wenn einzelne Komponenten nur in sehr geringen Mengen zum Einsatz gelangen, wie z.B. Enzyme, Coenzyme oder Chromogene. Ein Ausweg ist hier die Vermischung der geringen Mengen des Wirkstoffes mit einer größeren Menge an inerten Füllstoff und Verarbeitung des Gemisches zu einer Tablette, die dann leichter handhabbar ist.

Diese galenische Technik ist jedoch nicht problemlos in Bezug auf die Lösungszeit der Tablette und evtl. Wirkstoffverluste - z.B. Verlust an enzymatischer Aktivität - beim Verpressen des Gemisches zur Tablette. Sie setzt außerdem voraus, daß der Wirkstoff in fester Form haltbar zur Verfügung steht, was besonders bei Enzymen - z.B. Cholesterinoxidase - nicht immer gegeben ist.

Zur Lösung dieser Schwierigkeiten wurde vorgeschlagen, solche geringen Substanzmengen in einem Lösungsmittel aufzunehmen, eine Kapillare mit der entstehenden Lösung zu füllen, und die Substanz durch Verdunsten des Lösungsmittels in der Kapillare abzuscheiden. Eine solcherart beschichtete Kapillare stellt eine besonders vorteilhafte Darreichungsform der Reagenzkomponente dar, weil sie es ermöglicht, selbst geringste Substanzmengen ohne Zusatz von evtl. störenden Füllstoffen leicht zu handhaben. Ein Verfahren dieser Art ist in der DE-OS 27 21 942 beschrieben.

An die beschichteten Kapillaren sind eine Reihe von Forderungen zu stellen, deren Einhaltung für die prinzipielle Verwendbarkeit bzw. die Anwendungsbreite von wesentlicher Bedeutung sind. So muß die Substanz in haltbarer Form abgeschieden sein, ohne daß der Wirkstoff verdirbt. Das Verfahren muß außerdem eine gut reproduzierbare Beschichtung in Bezug auf die Substanzmenge gewährleisten und für eine industrielle Serienproduktion geeignet sein. Zuletzt ist es von Vorteil, wenn die Kapillare auch nach der Beschichtung noch saugfähig ist, um zur Dosierung weiterer Reagenzkomponenten - z.B. einer flüssigen Enzymsuspension - verwendet werden können.

Die Einhaltung dieser Forderungen stößt insbesondere auch bei einem wichtigen Chromogen der klinischen Chemie - 4-Aminophenazon - auf Schwierigkeiten. So neigt diese Substanz dazu, sich aus einer Vielzahl von Lösungsmitteln beim Verdunsten des Lösungsmittels nicht in kristalliner Form, sondern als amorphes Öl abzuscheiden. Das anfallende Öl beginnt sich nach kurzer Zeit unter Gelbfärbung zu verfestigen, wobei eine harzige hydrophobe Schicht entsteht, mit der die Kapillare ihre Saugfähigkeit verliert. Die Gelbfärbung der Substanz äußert sich in einer neuen Bande im Absorptionsspektrum und wirkt sich durch das Auftreten hoher Reagenzienleerwerte störend auf eine photometrische Messung aus. Variiert man die Bedingungen, unter denen das Lösungsmittel verdunstet, - z.B. durch Anlegen eines Unterdruckes - gelingt es zwar in Einzelfällen, eine feinkristalline Abscheidung mit guter Saugfähigkeit zu erreichen, es zeigt sich jedoch, daß diese Ergebnisse nur unsicher zu reproduzieren sind, so daß eine Serienproduktion damit nicht möglich ist.

Eine weitere Schwierigkeit ist dadurch gegeben, daß, selbst wenn eine kristalline Abscheidung mit prinzipiell guten Saugeigenschaften gelingt, sich die Substanz u.U. nicht in einer homogenen Schicht, sondern als Pfropfen an einem Kapillarende abscheiden kann, womit die Kapillare zugelegt wird und aus diesem Grunde nicht mehr saugfähig ist.

Das bekannte Verfahren der Gefriertrocknung ist nicht geeignet, die aufgezeigten Schwierigkeiten zu überwinden, weil es bei Einsatz von Kapillaren nur schwer möglich ist, den - für das Gelingen notwendigen - eingefrorenen Zustand während des gesamten Vorganges aufrecht zu erhalten. Aufgrund ihrer geringen Wärmekapazität neigen die Kapillaren einmal dazu, nach dem Einfrieren bereits auf dem Weg zur Gefriertrocknungsanlage wieder aufzutauen, zum anderen ist das Verhältnis von Wärmekapazität zu der dem Trockengut entzogene Sublimationsenergie so ungünstig, daß es ohne besondere Kühlung nicht gelingt, ein Auftauen des Kapillarinhaltes während der Trocknung selbst zu verhindern. Solche zusätzliche Kühlmaßnahme - wie z.B. die Verwendung einer gekühlten Stellfläche - sind jedoch wegen der ungünstigen Form der Kapillaren und des Umstandes, daß die beidseitig offen sind, nur schlecht anwendbar, so daß die Gefriertrocknung insgesamt gesehen keine Lösung bringt.

Der Erfindung liegt die Aufgabe zugrunde, die oben geschilderten Probleme zu beseitigen und ein einfaches Verfahren zu schaffen, welches ohne besonderen Aufwand eine homogene, kristalline und gut reproduzierbare Abscheidung von Chromogenen, insbesondere 4-Aminophenazon in Kapillaren unter Erhaltung der Saugfähigkeit ermöglicht und für eine Serienproduktion geeignet ist.

Gelöst wird diese Aufgabe erfindungsgemäß durch ein Verfahren mit den in Anspruch 1 angegebenen Maßnahmen.

Die anfängliche Übersättigung kann auf verschiedene Arten erreicht werden. In einer bevorzugten Ausführungsform des erfindungsgemäßen Verfahrens wird dabei ein Lösungsmittelgemisch aus einer das Chromogen lösenden Komponente und einer das Chromogen nicht lösenden Komponente verwendet, wobei eine vorgegebene Menge Chromogen mit gerade soviel der lösenden Komponente behandelt wird wie zur vollständigen Lösung notwendig ist und der Zustand der anfänglichen Übersättigung durch Hinzufügen einer geeigneten Menge der nicht lösenden Komponente erreicht wird.

Diese bevorzugte Ausführungsform weist einen besonderen Vorteil für die Anwendung in der klinischen Chemie auf. Meist wird dabei die Aufgabe bestehen, in einer Kapillare mit einem vorgegebenen Volumen eine ganz bestimmte Menge an Chromogen abzuscheiden, so daß zum Befüllen der Kapillare eine Lösung verwendet werden muß, die dieses Verhältnis von Chromogengehalt zu Volumen aufweist, d.h. die eine bestimmte Konzentration hat. Bei Verwendung eines Lösungsmittelgemisches der beschriebenen Art besteht nun im Gegensatz zu einem reinen Lösungsmittel die Möglichkeit, diese vorgegebene Konzentration in weiten Bereichen mit einen gewünschten Grad an Übersättigung zu verbinden, je nachdem, welche Menge der nicht lösenden Komponente zugesetzt wird.

Auch durch eine Senkung der Temperatur einer das Chromogen enthaltenden Lösung kann eine anfängliche Übersättigung erreicht und eine Auskristallisation ausgelöst werden. Von besonderem Vorteil kann insbesondere eine Kombination beider Maßnahmen sein, besonders dann, wenn aufgrund der anfänglichen Übersättigung schon während des Befüllens der Kapillare das Chromogen auszukristallisieren beginnt, d.h. die Lösung nicht handhabbar ist. In einer besonders bevorzugten Ausführungsform des erfindungsgemäßen Verfahrens wird deshalb durch Verwendung einer geeigneten Menge der nicht lösenden Komponente nur ein solcher Sättigungsgrad erreicht, daß die Lösung bei der Ansatztemperatur stabil oder metastabil, d.h. handhabbar ist, und durch anschließende Senkung der Temperatur eine Übersättigung erreicht, daß die Auskristallisierung beginnt.

Nach dem Auslösen der Kristallisation erfolgt der weitere Abscheidungsprozeß unter Verdunstung des Lösungsmittels. Dabei ist für das erfindungsgemäße Verfahren die Vermeidung einer Übersättigung von besonderer Bedeutung, denn überraschenderweise wurde gefunden, daß damit die beschriebene Pfropfenbildung an einem Kapillarende in einfacher Weise vermieden werden kann. Eine Übersättigung kann z.B. auftreten, wenn das Lösungsmittel so schnell verdunstet, daß nicht genügend Zeit für eine ungehinderte Kristallisation in einem der Verdunstungsgeschwindigkeit entsprechenden Ausmaß zur Verfügung steht. Mit fortschreitender Verdunstung bleibt dabei in der anfangs gefüllten Kapillare eine kurze Strecke einer stark übersättigten Lösung zurück, die sich aufgrund unvermeidbarer geringfügiger Schräglagerung der Kapillare an einem Ende ansammelt und aus der beim Verdunsten der letzten Anteile des Lösungsmittels der Pfropfen entsteht.

Wird für das erfindungsgemäße Verfahren ein Lösungsmittelgemisch verwendet, so ist darauf zu achten, daß die Dampfdrucke der einzelnen Komponenten aufeinander abgestimmt sind. Insbesondere muß vermieden werden, daß die nicht lösende Komponente leichter flüchtig ist als die lösende, weil sich damit eine starke Untersättigung ergeben würde, die ebenfalls zur Bildung eines Pfropfens führen muß.

Die Übersättigung kann auf verschiedene Arten vermieden werden, wobei es im wesentlichen darauf ankommt, die Verdunstung gerade so schnell ablaufen zu lassen, daß eine ungehinderte Kristallisation möglich ist.

So kann z.B. aus einer Reihe von chemisch homologen Lösungsmitteln mit im wesentlichen gleichen Lösungseigenschaften in Bezug auf das Chromogen dasjenige ausgewählt werden, bei dem die Verdunstungsgeschwindigkeit bei einer gegebenen Temperatur den erforderlichen Wert hat.

Zusätzlich zur Erreichung einer anfänglichen Übersättigung kann eine bestimmte Temperatur dazu dienen, die Verdunstungsgeschwindigkeit des Lösungsmittels oder Lösungsmittelgemisches so einzustellen, daß eine ungehinderte Kristallisation erreicht wird.

In an sich bekannter Weise kann die kristalline Abscheidung des Chromogens durch Zusatz von Kristallisationskeimen beschleunigt werden.

In der Praxis hat sich eine Kombination der vorgenannten Maßnahmen als zweckmäßig erwiesen. Bevorzugt wird dabei die Verwendung eines Lösungsmittelelgemisches - bestehend aus einer lösenden und einer nicht lösenden Komponente -,bei der die beiden Komponenten aus jeweils einer homologen Reihe so ausgewählt werden, daß die Verdunstungsgeschwindigkeit der lösenden Komponente geringfügig größer ist als die der nicht lösenden Komponente. Gleichzeitig wird durch eine Temperatur von -20 bis -50°C dafür gesorgt, daß die Verdunstung insgesamt nicht zu schnell verläuft.

Bei dieser bevorzugten Ausführungsform des Verfahrens werden außerdem in die Kapillaren vor dem Befüllen Kristallisationskeime, vorzugsweise feine Kristalle des Chromogens selbst, eingebracht.

Beispielsweise hat sich für die kristalline Abscheidung von 4-Aminophenazon als Lösungsmittelgemisch die Kombination eines Alkylalkohols als lösende und eines Dialkyläthers als nicht lösende Komponente als zweckmäßig erwiesen, wobei die Paarung Methanol und Diisopropyläther besonders vorteilhaft ist. Eine bei Raumtemperatur handhabbare d.h. metastabile übersättigte Lösung wird dabei erhalten, wenn die Lösung ca. 2.95 Vol% Methanol pro 100 µg 4-Aminophenazon enthält. Durch Einbringen von feinen 4-Aminophenazon-Kristallen in die leeren Kapillaren wird die Kristallisation nach dem Befüllen sofort ausgelöst, bei -50°C erfolgt die Verdunstung des Lösungsmittelgemisches gerade so schnell, daß kein Pfropfen entsteht. Bevorzugt erfolgt dabei die Abkühlung auf -50°C sehr schnell, d.h. ohne jede Zwischenlagerung der befüllten Kapillaren bei Raumtemperatur.

Prinzipiell muß nicht gewartet werden, bis das Lösungsmittel oder Lösungsmittelgemisch vollständig verdunstet und das Chromogen damit vollständig abgeschieden ist. Will man nur eine bestimmte Menge Chromogen abscheiden, so kann der Vorgang auch durch Absaugen des Flüssigkeitsrestes z.B. mit Filterpapier abgebrochen werden. Eine vollständige Verdunstung wird jedoch dann bevorzugt, wenn besondere Anforderungen an die Reproduzierbarkeit der abgeschiedenen Chromogenmenge von Kapillare zu Kapillare gestellt werden. Von besonderem Vorteil ist hier die bereits erwähnte Möglichkeit, jede gewünschte Menge an Chromogen durch geeignete Zusammensetzung des Lösungsmittelgemisches in eine beliebige anfängliche Übersättigung zu bringen.

Besonders bevorzugt ist weiterhin eine Ausführungsform des Verfahrens, bei der das Chromogen nach der Abscheidung zur Verbesserung der Haltbarkeit durch Anlegen eines Unterdruckes und ggf. Einsatz eines geeigneten Trockenmittels von den letzten Lösungsmittelresten befreit, d.h. nachgetrocknet wird.

Ein weiterer Vorteil des erfindungsgemäßen Verfahrens besteht darin, daß dabei homogene kristalline Schichten erreicht werden, die so gut an der Innenwand der Kapillaren haften, daß die abgeschiedene Substanz auch unter Einfluß starker mechanischer Erschütterungen nicht herausrieselt. Ergebnisse dieser Art lassen sich z.B. mit dem Verfahren der Gefriertrocknung - selbst wenn es trotz der geschilderten Schwierigkeiten durchführbar ist - nicht erreichen, weil die abgeschiedene Substanz dabei stets als lockerer Faden mit geringer Haftung an der Innenwand anfällt, der schon bei kleinen mechanischen Erschütterungen zerbröckelt und herausfällt. Das erfindungsgemäße Verfahren ist damit vorteilhaft überall da anwendbar, wo die beschichteten Kapillaren Erschütterungen dieser Art - wie sie z.B. auf dem normalen Versandweg zum Verbraucher unvermeidbar sind - unbeschädigt überstehen müssen.

Bei den in folgenden beschriebenen Anwendungsbeispielen dient zur Aufnahme der Kapillaren ein Kunststoffrad von ca. 200 mm Durchmesser, das am äußeren Umfang mit 48 Einfräßungen versehen ist, in denen die Kapillaren mit Hilfe eines um den Radumfang gespannten O-Ringes gehalten werden. Zur Handhabung des Rades dient ein runder Kunststoffstab von ca. 200 mm Länge, mit dem es über eine im Radzentrum vorgesehene Bohrung aufgenommen werden kann. Die verwendeten Glaskapillaren haben eine Länge von 32 mm und ein Volummen von 10 µl.

Die übersättigte 4-Aminophenazonlösung wird mittels einer Schlauchpumpe aus einem Vorratsbehälter in die Abfüllstation gepumpt, die aus einem flachen Durchflußbett besteht, aus dem die Lösung über eine Bohrung wieder abfließen kann und über einen Schlauch zurück in den Vorratsbehälter geleitet wird. Die Abschlußbohrung ist in einer bestimmten Höhe über dem Boden des Durchflußbettes angebracht, so daß in ihm ein konstanter Flüssigkeitspegel erhalten wird.

Beim Befüllen werden die auf dem Rad aufgespannten Kapillaren mit Hilfe des oben beschriebenen Aufnahmestabes unter einem Winkel von ca. 15 bis 30° schräg in das durchströmte Durchflußbett eingetaucht und durch langsames Drehen des Rades eine Kapillare nach der anderen befüllt.

Das Einbringen von Kristallisationskeimen in die Kapillaren - nachfolgend als "Bekeimung" bezeichnet - kann auf folgende Weise geschehen:

Fein gemahlenes 4-Aminophenazon wird in einer flachen Petrischale in einer Schichtdicke von ca. 2 mm ausgebreitet. Durch Eintauchen der auf dem Aufnahmerad eingespannten Kapillaren in diese Schicht wird in ihnen ein ca. 1 bis 1,5 mm langer Pfropfen von 4-Aminophenazon an einem Ende erzeugt, der anschließend durch Anblasen der Kapillaren an diesem Ende mit Preßluft gleichmäßig über die Innenwandung verteilt wird.

Diese mechanischen Hilfsmittel sind jedoch nicht eigentlicher Gegenstand des erfindungsgemäßen Verfahrens und bedürfen deswegen keiner besonderen Beschreibung.

Beispiel 1:

142 mg 4-Aminophenazon werden in 700 $\mu$l Methanol vollständig gelöst und die entstandene Lösung mit Di-sek-butyläther auf ein Volumen von 10ml gebracht. Nach Befüllen von ca. 8 mal 48 Kapillaren (8 Räder) werden die befüllten Kapillaren durch Einbringen in einen Kühlschrank einer Temperatur von -50°C ausgesetzt. Die Kristallisation von 4-Amoniphenazonen beginnt nach 15 Minuten. Nach 24 Stunden ist das Lösungsmittel vollständig verdunstet.

Zur Nachtrocknung werden die Kapillaren anschließend in einen Exsiccator eingebracht, in dem sich aktives Silicagel als Trockenmittel befindet, und es wird mit einer Öldrehschieberpumpe an den Exsiccator ein Unterdruck angelegt. Nach Erreichen des Endvakuums von ca. 1 mb wird die Pumpe abgeschaltet und die Kapillaren unter dem Einfluß des Trockenmittels 4 Stunden lang nachgetrocknet.

Beispiel 2:

292 mg 4- Aminophenazon werden in 860 µl Methanol gelöst und die entstandene Lösung mit Di-iso-propyläther auf 20 ml gebracht. Zum Befüllen werden ca. 15 mal 48 Kapillaren (15 Räder) verwendet, die vorher einer Bekeimung in der oben beschriebenen Weise unterzogen werden. Bei dieser Ausführungsform des Verfahrens beginnt die Auskristallisation von 4-Aminophenazon unmittelbar nach dem Befüllen der Kapillaren, so daß aus den geschilderten Gründen dafür Sorge getragen werden muß, daß sie ohne jede Zwischenlagerung bei Raumtemperatur durch Einbringen in den Kühlschrank sofort einer Temperatur von -50°C ausgesetzt werden. Nach zwei Stunden ist das Lösungsmittel vollständig verdunstet, die Nachtrocknung erfolgt in der im Beispiel 1 beschriebenen Weise:

Zur Bestimmung des Gehaltes der beschichteten Kapillaren kann folgendes Verfahren dienen:

Von jedem Rad werden 5 bis 10 Kapillaren entsprechend einem Stichprobenumfang von 10,4 bis 20,8 % entnommen und jeweils in ein Reagenzglas mit 10 ml destilliertem Wasser eingebracht. Durch kräftiges Schütteln wird die abgeschiedene Substanz in Lösung gebracht und die Extinktion der entstehenden Lösung bei einer Wellenlänge von 270 mm photometrisch gegen destilliertes Wasser gemessen.

Das erfindungsgemäße Verfahren führt zu zufriedenstellenden Ergebnissen hinsichtlich einer Serienproduktion. Beispielsweise wurden mit der angegebenen Prüfmethode bei den Ausführungsformen gemäß Beispiel 1 und 2 folgende typische Meßwerte gefunden:

|  | Beispiel 1 | Beispiel 2 |
|---|---|---|
| Stichprobenumfang | 20,8 % | 10,4 % |
| Mittelwert der Extinktion | 0,55 | 0,58 |
| Standardabweichung | 0,02 | 0,017 |
| Variationskoeffizient | 3,6 % | 2,8 % |

Die Einzelmessungen genügen jeweils einer Normalverteilung. Die Prüfung der Saugfähigkeit mit einer Enzymsuspension, enthaltend 4000 Einheiten Cholesterinoxidase pro Liter, 12 000 Einheiten Cholesterinesterase pro Liter und 16 000 Einheiten Peroxidase pro Liter, ergibt Saugzeiten von weniger als 7 sec. (Beispiel 1, Stichprobenumfang 20,8 %) bzw. weniger als 4 sec. (Beispiel 2, Stichprobenumfang 10,4 %).

Compur-Electronic GmbH　　　　München, den 4. April 1979
München　　　　　　　　　　770/Dr.G/Ve/DP-753


Verfahren zur kristallinen Abscheidung
von Chromogenen
_____

Patentansprüche:
_____


1. Verfahren zur kristallinen Abscheidung von Chromogenen
in Kapillaren, insbesondere für klinisch-chemische Bestimmungen, d a d u r c h  g e k e n n z e i c h n e t ,
 - daß die Auskristallisation durch eine anfängliche
   Übersättigung ausgelöst wird und
 - daß die Lösung während des gesamten weiteren Kristallisationsvorganges bei Verdunsten des Lösungsmittels

0017812

2. Verfahren nach Anspruch 1, dadurch gekennzeichnet, daß aus einer Reihe von chemisch homologen Lösungsmitteln mit im wesentlichen gleichen Lösungseigenschaften dasjenige durch Substitution ausgewählt wird, bei dem die Lösung während des gesamten weiteren Kristallisationsvorganges nicht mehr in einen übersättigten Zustand gelangt.

3. Verfahren nach Anspruch 1 oder 2, dadurch gekennzeichnet, daß als Lösungsmittel ein Lösungsmittelgemisch verwendet wird.

4. Verfahren nach einem der Ansprüche 1 bis 3, dadurch gekennzeichnet, daß das Lösungsmittelgemisch aus einer das Chromogen lösenden und einer das Chromogen im wesentlichen nicht lösenden Komponente besteht.

5. Verfahren nach einem der Ansprüche 1 bis 4, dadurch gekennzeichnet, daß soviel von der Chromogen lösenden Komponente verwendet wird wie zum Lösen einer vorgegebenen Menge an Chromogen mindestens notwendig ist, und daß der Zustand der anfänglichen Übersättigung durch Zusatz einer geeigneten Menge der nicht lösenden Komponente erreicht wird.

6. Verfahren nach einem der Ansprüche 1 bis 5, dadurch gekennzeichnet, daß als lösende Komponente ein Äther verwendet wird.

7. Verfahren nach einem der Ansprüche 1 bis 6, dadurch gekennzeichnet, daß als Chromogen 4-Aminophenazon, als nicht lösende Komponente ein Dialkyläther und als lösende Komponente Methanol verwendet wird.

- 3 -

0017812

8. Verfahren nach einem der Ansprüche 1 bis 7, dadurch gekennzeichnet, daß als Dialkyläther ein symmetrischer Dialkyläther mit 1 bis 5 C-Atomen pro Seitenkette verwendet wird.

9. Verfahren nach einem der Ansprüche 1 bis 8, dadurch gekennzeichnet, daß als Dialkyläther Diisopropyläther verwendet wird.

10. Verfahren nach einem der Ansprüche 1 bis 9, dadurch gekennzeichnet, daß als lösende Komponente 2 bis 6 Vol% Methanol pro 100 µg 4-Aminophenazon verwendet wird.

11. Verfahren nach einem der Ansprüche 1 bis 10, dadurch gekennzeichnet, daß 2.95 Vol% Methanol pro 100 µg 4-Aminophenazon verwendet wird.

12. Verfahren nach einem der Ansprüche 1 bis 11, dadurch gekennzeichnet, daß die Temperatur des Lösungsmittels zu dessen Verdunstung so gewählt wird, daß eine Auskristallisation erfolgt, jedoch eine Übersättigung vermieden wird.

13. Verfahren nach einem der Ansprüche 1 bis 12, dadurch gekennzeichnet, daß zur Vermeidung einer Übersättigung beim Kristallisationsvorgang die Lösung auf eine Temperatur gebracht wird, bei der das Lösungsmittel gerade so schnell verdunstet, daß eine ungehinderte Kristallisation erreicht wird.

14. Verfahren nach einem der Ansprüche 1, bis 13, dadurch gegekennzeichnet, daß die Temperatur kleiner als -30°C ist.

15. Verfahren nach einem der Ansprüche 1 bis 14, dadurch gekennzeichnet, daß die Temperatur kleiner oder gleich -50°C ist.

- 4 -

0017812

16. Verfahren nach einem der Ansprüche 1 bis 15, dadurch gekennzeichnet, daß die Temperatur so lange aufrecht erhalten wird, bis das Lösungsmittel vollständig verdunstet ist.

17. Verfahren nach einem der Ansprüche 1 bis 16, dadurch gekennzeichnet, daß nach dem Abscheiden des Chromogens eine Nachtrocknung unter Anlegen eines Unterdrucks erfolgt.

18. Verfahren nach einem der Ansprüche 1 bis 17, dadurch gekennzeichnet, daß in die Kapillaren vor dem Befüllen mit der das Chromogen enthaltenden Lösung Kristallisationskeime eingebracht werden.

19. Verfahren nach einem der Ansprüche 1 bis 18, dadurch gekennzeichnet, daß als Kristallisationskeime feine Kristalle des abzuscheidenden Chromogens verwendet werden.

# EUROPÄISCHER RECHERCHENBERICHT

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| | DE – A1 – 2 831 083 (GIST-BROCADES) <br> * Ansprüche 8, 9 * <br> -- | 1,16 |
| A | DE – A1 – 2 746 766 (RADIOMETER) <br> * Seiten 11 bis 13 * <br> -- | 1 |
| A,D | DE – A1 – 2 721 942 (BAYER) <br> * ganzes Dokument * <br> -- | |
| A,D | DE – A1 – 2 422 260 (COMPUR-WERK) <br> * ganzes Dokument * <br> -- | |
| A | DE – A1 – 2 639 176 (UNIVERSITY OF DELAWARE) <br> * ganzes Dokument * <br> ---- | 18 |

**KLASSIFIKATION DER ANMELDUNG (Int.Cl.3)**

G 01 N 33/52
B 01 D 9/00

**RECHERCHIERTE SACHGEBIETE (Int. Cl.3)**

B 01 D 9/00
G 01 N 33/48

**KATEGORIE DER GENANNTEN DOKUMENTE**

X: von besonderer Bedeutung

A: technologischer Hintergrund

O: nichtschriftliche Offenbarung

P: Zwischenliteratur

T: der Erfindung zugrunde liegende Theorien oder Grundsätze

E: kollidierende Anmeldung

D: in der Anmeldung angeführtes Dokument

L: aus andern Gründen angeführtes Dokument

&: Mitglied der gleichen Patent- familie, übereinstimmendes Dokument

Der vorliegende Recherchenbericht wurde für alle Patentansprüche erstellt.

| Recherchenort | Abschlußdatum der Recherche | Prüfer |
|---|---|---|
| Berlin | 09-07-1980 | SCHWARTZ |

EPA form 1503.1 08.78